(19) Europäisches Patentamt
European Patent Office
Office européen des brevets

(11) Veröffentlichungsnummer: **0 011 706**
**B1**

# (12) EUROPÄISCHE PATENTSCHRIFT

(45) Veröffentlichungstag der Patentschrift: **03.03.82**

(21) Anmeldenummer: **79104044.7**

(22) Anmeldetag: **19.10.79**

(51) Int. Cl.³: **C 07 D 211/90,** C 07 D 401/04, A 61 K 31/44 // (C07D401/04, 211/90, 213/04)

## (54) Verfahren zur Herstellung von teilweise neuen 1,4-Dihydropyridin-3-carbonsäure-Derivaten (I), neue 1,4-Dihydropyridin-3-carbonsäure-Derivate (II) und die Verwendung der Derivate (I) und (II) zur Herstellung von 1,4-Dihydropyridin-3,5-dicarbonsäurediestern.

(30) Priorität: **31.10.78 DE 2847237**
**26.05.79 DE 2921429**

(43) Veröffentlichungstag der Anmeldung: **11.06.80 Patentblatt 80/12**

(45) Bekanntmachung des Hinweises auf die Patenterteilung: **03.03.82 Patentblatt 82/9**

(84) Benannte Vertragsstaaten: **CH DE FR GB**

(56) Entgegenhaltungen:
**DE-A-2 218 644**
**Chemical Abstracts, Vol:83 (1975)**
**+83: 178731r+**

(73) Patentinhaber: **BAYER AG, Zentralbereich Patente, Marken und Lizenzen, D-5090 Leverkusen 1, Bayerwerk (DE)**

(72) Erfinder: **Wehinger, Egbert, Dr., Donnenbergerstrasse 90, D-5620 Velbert 15 (DE)**
Erfinder: **Bossert, Friedrich, Dr., Claudiusweg 7, D-5600 Wuppertal (DE)**

EP 0 011 706 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von teilweise neuen 1,4-Dihydropyridin-3-carbonsäure-Derivaten (I), neue 1,4-Dihydropyridin-3-carbonsäure-Derivate (II) und die Verwendung der Derivate (I) und (II) zur Herstellung von 1,4-Dihydropyridin-3,5-dicarbonsäurediestern

Die vorliegende Erfindung betrifft ein neues Verfahren zur Herstellung von teilweise neuen 1,4-Dihydropyridin-3-carbonsäure-derivaten (I), neue 1,4-Dihydropyridin-3-carbonsäure-Derivate (II) und die Verwendung der Derivate (I) und (II) zur Herstellung von 1,4-Dihydropyridin-3,5-dicarbonsäurediestern. Die erfindungsgemässen Verbindungen und die aus ihnen hergestellten Diester besitzen kreislaufbeeinflussende Wirkungen und können bei der Herstellung von Arzneimitteln eingesetzt werden.

Es ist bereits bekannt geworden, dass man N-Aryl-substituierte Dihydropyridin-3,5-dicarbonsäureester unter Einwirkung von Alkalien in entsprechende Dihydropyridinmono- bzw. -dicarbonsäuren überführen kann (vgl. Br. Lachowicz, Monatsh. Chem. 17, 343 (1896) ).

Weiterhin ist bekannt geworden, dass N-Alkylsubstituierte Dihydropyridinmonocarbonsäuren aus den entsprechenden 3,5-Diestern in ähnlicher Weise durch alkalische Hydrolyse zugänglich sind (vgl. A. E. Sausin et al, Gieterotsiklich Soedin 1978, (2) 272).

Im Gegensatz zu der leichten Verseifbarkeit von N-Aryl- und N-Alkyl-substituierten Dihydropyridin-3,5-dicarbonsäurediestern lassen sich N-unsubstituierte Dihydropyridindicarbonsäurediester entweder gar nicht oder nur in sehr schlechten Ausbeuten zu den entsprechenden Mono- bzw. Dicarbonsäuren hydrolysieren (vgl. U. Eisner et al, Chem. Rev. 72, 1, 41 (1972); B. Loev et al, J. Heterocyclic Chem. 12, 363 (1975) ).

Aus dem Stand der Technik ist bekannt, dass elektronenziehende Gruppen die alkalische Esterhydrolyse erleichtern. Dies gilt auch für solche Dihydropyridincarbonsäureester, die am Stickstoff in 1-Position eine Aryl- oder Alkyl-Substituenten tragen. Die Hydrolyse von Dihydropyridincarbonsäureestern mit unsubstituierter 1-Position war bisher nicht in befriedigender Weise durchführbar. B. Loev et al., J. Heterocyclic Chem. 12, 363–365 (1975) beschreibt die Hydrolysebedingungen für Dihydropyridinester und auch A. Phillips, J. Am. Chem. Soc. 71, 4003 (1949) hat die Hydrolyse von Dihydropyridinen, die in 4-Position einen Chinolinrest tragen, untersucht. Im Ergebnis stimmen beide darin überein, dass eine Hydrolyse der Estergruppen unter den angegebenen Bedingungen praktisch nicht möglich ist.

Überraschenderweise wurde gefunden, dass bei der Verwendung von Dihydropyridinestern, die eine 2-Cyanoethyl-estergruppe tragen, alkalische Verseifung auch solcher Dihydropyridine, die in 1-Position unsubstituiert sind, bzw. die in 4-Position einen Chinolinrest tragen, mit guten Ausbeuten durchführbar ist.

Es wurde gefunden, dass man die teilweise neuen N-unsubstituierten 1,4-Dihydropyridin-3-carbonsäure-derivate der allgemeinen Formel

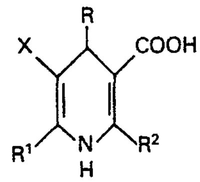

(I)

in welcher

R für einen Pyridylrest oder für einen Phenylrest steht, der ein oder zwei gleiche oder verschiedene Substituenten aus der Gruppe Halogen, Nitro, Cyano, Trifluormethyl, Azido, Trifluormethoxy oder Alkoxy mit 1 bis 2 Kohlenstoffatomen, enthält

$R^1$ und $R^2$ gleich oder verschieden sind und für Wasserstoff, eine Alkyl-Gruppe mit 1 bis 2 Kohlenstoffatomen, einen Phenyl- oder Benzyl-Rest stehen und

X a) für die Gruppe $-COOH$ oder

b) für die Gruppe $-COR^3$, wobei $R^3$ Alkyl mit 1 bis 4 Kohlenstoffatomen bedeutet, oder

c) für die Gruppe $-COOR^4$, wobei $R^4$ einen geradkettigen, verzweigten oder cyclischen, gesättigten oder ungesättigten Kohlenwasserstoffrest, der gegebenenfalls durch ein Sauerstoff- oder Schwefelatom oder durch die $-SO_2$-Gruppe in der Kette unterbrochen ist, oder durch Trifluormethyl oder durch den Rest

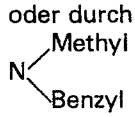 substituiert ist, oder einen Benzylrest

darstellt, oder

d) für die Gruppe $-SO_2-R^5$ steht, wobei $R^5$ einen geradkettigen oder verzweigten, gesättigten oder ungesättigten Kohlenwasserstoffrest mit bis zu 6 Kohlenstoffatomen oder einen Phenylrest bedeutet, erhält, wenn man 1,4-Dihydropyridin-derivate der allgemeinen Formel II

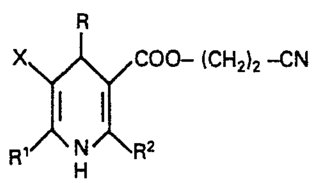

(II)

in welcher R, $R^1$, $R^2$ und X die oben angegebene Bedeutung haben, in Gegenwart von inerten organischen Lösungsmitteln und Wasser in einem Temperaturbereich von 10 bis 100 °C alkalisch hydrolisiert.

Es ist ausgesprochen überraschend, dass nach dem erfindungsgemässen Verfahren die 1,4-Dihydropyridincarbonsäuren der allgemeinen Formel I in so guten Ausbeuten und hoher Reinheit erhalten werden, weil man im Hinblick auf den Stand der Technik erwarten musste, dass die N-unsubstituierten 1,4-Dihydropyridincarbonsäuren unter diesen Hydrolysebedingungen entweder gar nicht oder nur in sehr schlechten Ausbeuten zu erhalten

sind (vgl. B. Loev et al, J. Heterocyclic Chem. 12, 363 (1975) ). Durch das Verfahren der vorliegenden Erfindung wird somit ein bestehendes Vorurteil gegen die Hydrolysierbarkeit von Dihydropyridincarbonsäureestern überwunden.

Ein wesentlicher Vorteil des Verfahrens liegt neben den guten Ausbeuten und der hohen Reinheit des erhaltenen Produktes darin, dass es aufgrund seiner einfachen Reaktionsbedingungen mit geringem technischem Aufwand und hoher Wirtschaftlichkeit durchgeführt werden kann.

In der DT-OS 2 218 644 werden bereits einige Dihydropyridin-monocarbonsäuren als Ausgangsstoffe für eine Estersynthese erwähnt, ebenso wie ihre prinzipielle Herstellbarkeit durch alkalische Hydrolyse von entsprechenden Diestern. Bei den meisten der dort erwähnten Dihydropyridin-monocarbonsäuren handelt es sich jedoch um solche, die am Stickstoff in 1-Position substituiert sind. Physikalisch-chemische Parameter und Ausbeuteangaben bzw. konkrete Verfahrensmassnahmen für die Hydrolyse sind dort jedoch nicht offenbart.

Wie bereits in den Ausführungen zum Stand der Technik dargelegt, konnten die Dihydropyridindicarbonsäurediester bisher nur dann problemlos verseift werden, wenn sie am Stickstoff in 1-Position einen Substituenten besassen. Die Verseifung von N-unsubstituierten Dihydropyridinen gelang bisher gar nicht oder nur mit so geringen Ausbeuten, dass eine technische Durchführung des Verfahrens uninteressant erschien.

Überraschenderweise ermöglicht die Einführung einer elektronenziehenden Gruppe im abzuspaltenden Esterrest eine einfache Verseifung zu den bisher schwer zugänglichen, N-unsubstituierten Monocarbonsäuren der Formel I.

Die erfindungsgemäss hergestellten Dihydropyridincarbonsäuren der allgemeinen Formel I besitzen selbst wertvolle pharmakologische Eigenschaften. Aufgrund ihrer kreislaufbeeinflussenden Wirkung können sie z.B. als antihypertensive Mittel, als periphere und zerebrale Vasodilatatoren sowie als Coronartherapeutika Verwendung finden.

Darüberhinaus sind sie besonders geeignet als Ausgangsstoffe für die Herstellung von Dihydropyridincarbonsäure-diestern, die ihrerseits wertvolle pharmakologische Eigenschaften, insbesondere kreislaufbeeinflussende Wirkung zeigen. Die Herstellung dieser Diester erfolgt üblicherweise nach bekannten Veresterungsmethoden, zum Beispiel durch Umsetzung nach der Dicyclohexylcarbodiimid-Methode (DCC-Methode) (vgl. Angew. Chem. 90, 556 ff (1978) ) oder durch Umsetzung der entsprechenden Dihydropyridincarbonsäure-imidazolide mit Alkoholen (vergl. H. A. Staab et al. Neuere Methoden der präparativen Organ. Chemie Vol. V, Seite 53 ff (1967) ).

Die erfindungsgemäss hergestellten 1,4-Dihydropyridincarbonsäuren, insbesondere die bisher noch nicht bekannten Vertreter dieser Stoffklasse, stellen wertvolle Ausgangsstoffe dar, aus denen sich

a) Dihydropyridin-3,5-dicarbonsäureester mit nicht identischen Esterfunktionen von hoher Reinheit unter milden Bedingungen darstellen lassen, indem die zweite Alkohol-Esterkomponente spezifisch in das Molekül eingeführt wird, und

b) markierte Dihydropyridindiester in einfacher Weise darstellen lassen, durch Einführung eines durch Deuterium oder Tritium markierten Alkoholrestes wie z.B. eines pertritiierten Methylatrestes, die für Metabolismusuntersuchungen von Arzneimittelwirkstoffen von besonderer Bedeutung sind und damit die Herstellung dieser markierten Verbindungen in einer Stufe erlaubt.

Verwendet man 1,4-Dihydro-2,6-dimethyl-4-(3'-nitrophenyl)-pyridin-3,5-dicarbonsäure-(2-cyanoäthyl)-isopropylester als Ausgangsstoff, so kann der Reaktionsablauf durch folgendes Formelschema wiedergegeben werden:

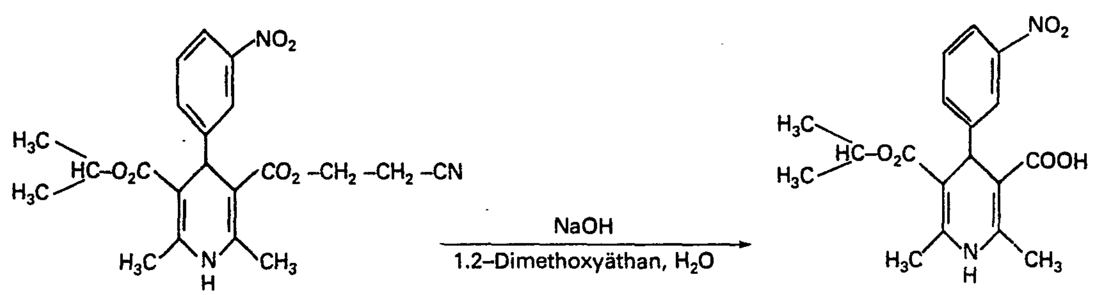


Nach dem erfindungsgemässen Verfahren wird ein 1,4-Dihydropyridin-Derivat der allgemeinen Formel II vorzugsweise in Gegenwart eines mit Wasser mischbaren organischen Lösungsmittels bei Temperaturen zwischen 20 und 50 °C alkalisch hydrolysiert.

Von besonderem Interesse ist die Herstellung von Verbindungen der allgemeinen Formel (I), in welcher

R für einen 3-Nitrophenylrest, einen 2-Trifluormethylphenylrest, einen 2-Chlorphenylrest oder einen 2-Methylmercaptopyridylrest steht,

$R^1$ und $R^2$ für Methyl stehen und

X für die Gruppe –COOH oder $COOR^4$ steht, wobei $R^4$ für einen geradkettigen, verzweigten oder cyclischen Alkylrest mit bis zu 6 Kohlenstoffatomen, der gegebenenfalls durch ein Sauerstoffatom in der Kette unterbrochen oder durch 1 bis 2 Trifluormethylgruppen oder durch den Rest

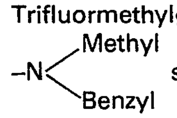
substituiert ist, steht.

Als Ausgangsverbindungen werden vorzugsweise solche Dihydropyridine der allgemeinen Formel II eingesetzt, in welcher

R, $R^1$, $R^2$ und X die oben angegebene Bedeutung haben,

n für die Zahl 2 steht und

Y für Fluor, Chlor, Cyano oder für eine Estergruppe der Formel $-COOR^6$, wobei $R^6$ Alkyl mit 1 bis 2 Kohlenstoffatomen oder Benzyl bedeutet, oder für die Gruppe $-O-CO-R^7$ steht, wobei $R^7$ Alkyl mit 1 bis 2 Kohlenstoffatomen oder Phenyl bedeutet.

Die als Ausgangsstoffe verwendeten 1,4-Dihydropyridin-Derivate der allgemeinen Formel II sind bekannt oder können in an sich bekannter Weise durch Umsetzung von Ylidencarbonylverbindungen mit Enaminocarbonsäureester oder durch Umsetzung von Aldehyden mit Enaminoverbindungen und β-Ketocarbonsäureestern erhalten werden (vgl. DT-OS 2 117 571, Brit. Pat. 1 358 951, DT-OS 2 117 572, Brit. Pat. 1 331 405).

Als Beispiele seien genannt:

1,4-Dihydro-2,6-dimethyl-4-(2'-nitrophenyl)-pyridin-3,5-dicarbonsäure-methyl-(2-cyanoäthyl)-ester;

1,4-Dihydro-2,6-dimethyl-4-(3'-nitrophenyl)-pyridin-3,5-dicarbonsäure-butyl-(2-cyanoäthyl)-ester;

1,4-Dihydro-2,6-dimethyl-4-(3'-nitrophenyl)-pyridin-3,5-dicarbonsäure-isopropyl-(2-cyanoäthyl)-ester;

1,4-Dihydro-2,6-dimethyl-4-(3'-nitrophenyl)-pyridin-3,5-dicarbonsäure-cyclopentyl-(2-cyanoäthyl)-ester;

1,4-Dihydro-2,6-dimethyl-4-(3'-nitrophenyl)-pyridin-3,5-dicarbonsäure-allyl-(2-cyanoäthyl)-ester;

1,4-Dihydro-2,6-dimethyl-4-(3'-nitrophenyl)-pyridin-3,5-dicarbonsäure-(2-methoxyäthyl)-(2-cyanoäthyl)-ester;

1,4-Dihydro-2,6-dimethyl-4-(3'-nitrophenyl)-pyridin-3,5-dicarbonsäure-(2-methylthioäthyl)-(2-cyanoäthyl)-ester;

1,4-Dihydro-2,6-dimethyl-4-(3'-nitrophenyl)-pyridin-3,5-dicarbonsäure-benzyl-(2-cyanoäthyl)-ester;

1,4-Dihydro-2,6-dimethyl-4-(3'-nitrophenyl)-pyridin-3,5-dicarbonsäure-(3,4-dichlorbenzyl)-(2-cyanoäthyl)-ester;

1,4-Dihydro-2,6-dimethyl-4-(3'-nitrophenyl)-pyridin-3,5-dicarbonsäure-(2,2,2-trifluoräthyl)-(2-cyanoäthyl)-ester;

1,4-Dihydro-2,6-dimethyl-4-(2'-trifluormethylphenyl)-pyridin-3,5-dicarbonsäure-äthyl-(2-cyanoäthyl)-ester;

1,4-Dihydro-2,6-dimethyl-4-(3'-cyanophenyl)-pyridin-3,5-dicarbonsäure-isopropyl-(2-cyanoäthyl)-ester;

1,4-Dihydro-2,6-dimethyl-4-(2'-methoxyphenyl)-pyridin-3,5-dicarbonsäure-methyl-(2-cyanoäthyl)-ester;

1,4-Dihydro-2,6-dimethyl-4-(3'-chlorphenyl)-pyridin-3,5-dicarbonsäure-isopropyl-(2-cyanoäthyl)-ester;

1,4-Dihydro-2,6-dimethyl-4-(2'-fluorphenyl)-pyridin-3,5-dicarbonsäure-isopropyl-(2-cyanoäthyl)-ester;

1,4-Dihydro-2,6-dimethyl-4-(2-pyridyl)-pyridin-3,5-dicarbonsäure-isopropyl-(2-cyanoäthyl)-ester;

1,4-Dihydro-2,6-dimethyl-4-(3-pyridyl)-pyridin-3,5-dicarbonsäure-isopropyl-(2-cyanoäthyl)-ester;

Als Hydrolyseagentien kommen in erster Linie anorganische Basen in Betracht. Hierzu gehören vorzugsweise Alkalihydroxide wie Natriumhydroxid oder Kaliumhydroxid. Die Basen können je nach Art der organischen Ausgangsverbindung in molaren Mengen oder in zwei- bis dreifachem Überschuss eingesetzt werden.

Als Reaktionsmedium hat sich ein grosser Überschuss an Wasser als vorteilhaft erwiesen. Zur homogenen Reaktionsführung ist es in der Regel zweckmässig, ein inertes, mit Wasser mischbares organisches Lösungsmittel zuzugeben. Hierzu gehören vorzugsweise Alkohole wie Methanol, Äthanol oder Propanol, Äther wie Dioxan, Tetrahydrofuran oder 1,2-Dimethoxyäthan, oder Pyridin, Dimethylformamid, Dimethylsulfoxid oder Hexamethylphosphorsäuretriamid.

Besonders vorteilhaft hat sich die Verwendung von aliphatischen Alkoholen mit 1 bis 4 Kohlenstoffatomen und 1,2-Dimethoxyäthan als Lösungsmittel bewährt.

Die Reaktionstemperaturen können in einem grösseren Bereich variiert werden, im allgemeinen arbeitet man zwischen 10 und 100 °C, insbesondere zwischen 20 und 50 °C. Vorzugsweise arbeitet man bei Raumtemperatur.

Die Umsetzung kann bei Normaldruck, aber auch bei erhöhtem Druck durchgeführt werden. In der Regel arbeitet man bei Normaldruck.

Bei der Durchführung des erfindungsgemässen Verfahrens wird 1 Mol der organischen Ausgangsverbindung der Formel II mit 1 bis 3 Mol einer anorganischen Base in einem geeigneten wässrig organischen Lösungsmittelgemisch zur Reaktion gebracht. Anschliessend wird die Mischung mit Wasser verdünnt und mit Methylenchlorid extrahiert. Die wässrige Phase wird abgetrennt und sauer gestellt, wobei die erfindungsgemässen Reaktionsprodukte ausfallen. Diese werden abgesaugt und aus einem inerten organischen Lösungsmittel umkristallisiert.

Die erfindungsgemäss hergestellten Verbindungen und die aus ihnen herstellbaren Diester haben ein breites und vielseitiges pharmakologisches Wirkungsspektrum.

1. Die Verbindungen bewirken bei parenteraler, oraler und perlingualer Zugabe eine deutliche und langanhaltende Erweiterung der Coronargefässe. Diese Wirkung auf die Coronargefässe wird durch einen gleichzeitigen Nitritähnlichen herzentlastenden Effekt verstärkt.

Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so dass eine in therapeutischen Dosen nachweisbare Antiflimmerwirkung resultiert.

3. Der Tonus der glatten Muskulatur der Gefässe wird unter der Wirkung der Verbindungen stark vermindert. Diese gefässspasmolytische Wirkung kann im gesamten Gefässsystem stattfinden, oder sich mehr oder weniger isoliert in umschriebenen

Gefässgebieten (wie z.B. dem Zentralnervensystem) manifestieren.

4. Die Verbindungen senken den Blutdruck von normotonen und hypertonen Tieren und können somit als antihypertensive Mittel verwendet werden.

5. Die Verbindungen haben stark muskulärspasmolytische Wirkungen, diese an der glatten Muskulatur des Magens, Darmtraktes, des Urogenitaltraktes und des Respirationssystems deutlich werden.

Die erfindungsgemäss hergestellten Verbindungen und die aus ihnen erhältlichen Diester können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel, wie Paraffine (z.B. Erdölfraktionen), pflanzliche Öle (z.B. Erdnuss/Sesamöl), Alkohole (z.B. Äthylalkohol, Glycerin), Glykole (z.B. Propylenglykol, Polyäthylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaufen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich ausser den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergl. enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Einige der erfindungsgemäss herstellbaren Verbindungen sind neu. Sie werden ebenfalls durch die vorliegende Anmeldung umfasst. Von besonderem Interesse sind die folgenden neuen Verbindungen der allgemeinen Formel I in welcher

R für einen 3-Nitrophenylrest, einen 2-Trifluormethylphenylrest, einen 2-Chlorphenylrest oder einen 2-Methylmercaptopyridylrest steht,

$R^1$ und $R^2$ für Methyl stehen und

X für die Gruppe –COOH oder $-COOR^4$ steht, wobei $R^4$ für einen geradkettigen, verzweigten oder cyclischen Alkylrest mit bis zu 6 Kohlenstoffatomen, der gegebenenfalls durch ein Sauerstoffatom in der Kette unterbrochen oder durch 1 bis 2 Trifluormethylgruppen oder durch den Rest

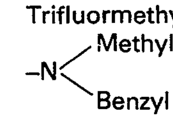
substituiert ist, steht.

Ebenfalls von Interesse sind die neuen Verbindungen der Formel I, in welcher

R für einen 2-Nitrophenylrest steht,

$R^1$ und $R^2$ jeweils Methyl bedeuten und

X für $COOR^4$ steht, wobei $R^4$ für einen Methyl- oder einen Isobutylrest steht.

Diese neuen Verbindungen sind vorzugsweise geeignet als Ausgangsprodukte zur Herstellung von besonders vorteilhaft wirksamen Dihydropyridinderivaten, von reinen unsymmetrischen Diestern und von markierten Diestern, insbesondere von Dihydropyridindiestern, die übliche Veresterungsmethoden aus Verbindungen der Formel I hergestellt werden.

Beispiel 1
1,4-Dihydro-2,6-dimethyl-4-(3'-nitrophenyl)-pyridin-3,5-dicarbonsäuremonoisopropylester

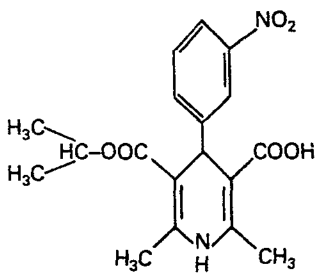

41,3 g (0,1 Mol) 1,4-Dihydro-2,6-dimethyl-4-(3'-nitrophenyl)-pyridin-3,5-dicarbonsäure-5-isopropyl-3-(2-cyanoäthyl)-ester wurden in einer Lösung von 12 g (0,3 Mol) Natriumhydroxid in 300 ml Wasser plus 150 ml 1,2-Dimethoxyäthan 7 Stunden bei Raumtemperatur gerührt. Anschliessend wurde die Reaktionsmischung mit 100 ml Wasser verdünnt und mehrmals mit Methylenchlorid extrahiert. Die organischen Extrakte wurden verworfen, die wässrige Phase mit verdünnter Salzsäure sauer gestellt. Dabei fiel das Reaktionsprodukt aus. Dieses wurde abgesaugt und aus Methanol umkristallisiert.

Schmelzpunkt: 182 bis 184 °C (Zers.), Ausbeute: 26 g (72%)

Analog Beispiel 1 wurden folgende Verbindungen erhalten (Tabelle 1):

Tabelle 1

| Nr. | Formel | Lösungsmittel | Schmelzpunkt | Ausbeute |
|---|---|---|---|---|
| 2 | 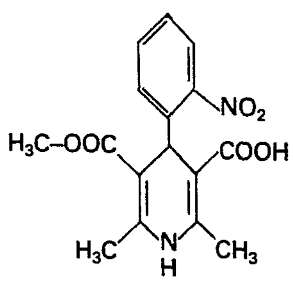 | 1,2-Dimethoxyäthan Wasser | 186 °C (Zers.) | 42% |
| 3 | 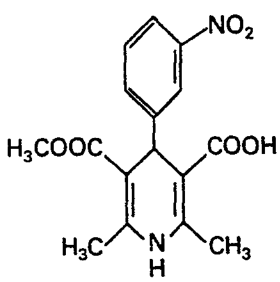 | 1,2-Dimethoxyäthan Wasser | 203 °C (Zers.) | 81% (Rohprodukt) |
| 4 | 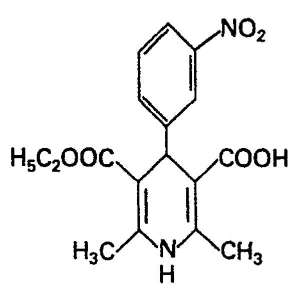 | 1,2-Dimethoxyäthan Wasser | 191 °C (Zers.) | 41% |
| 5 | 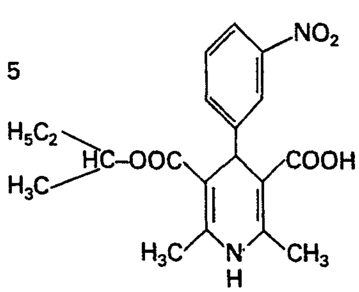 | 1,2-Dimethoxyäthan Wasser | 153 °C (Zers.) | 60% |

Tabelle 1 (Fortsetzung)

| Nr. | Formel | Lösungsmittel | Schmelzpunkt | Ausbeute |
|---|---|---|---|---|
| 6 | 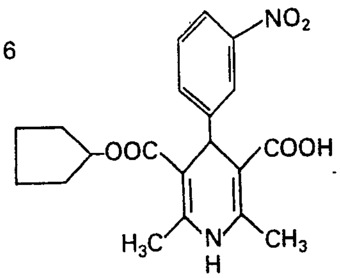 | 1,2-Dimethoxyäthan Wasser | 208 °C (Zers.) | 65% |
| 7 | 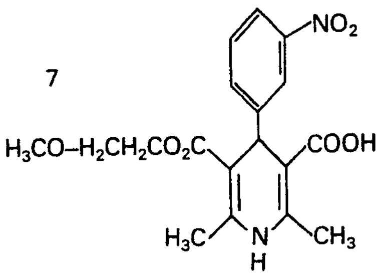 | 1,2-Dimethoxyäthan Wasser | 192 °C (Zers.) | 56% |
| 8 | 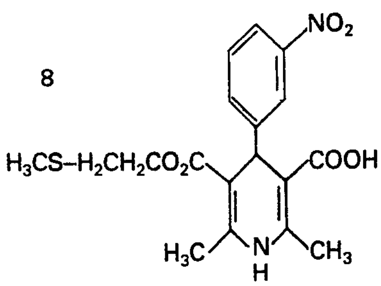 | 1,2-Dimethoxyäthan Wasser | 203 °C (Zers.) | 32% |
| 9 | 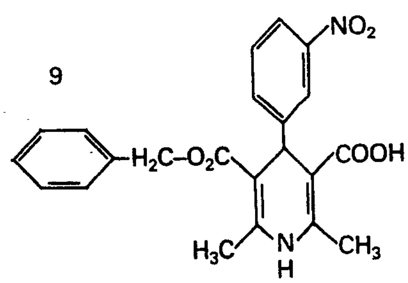 | 1,2-Dimethoxyäthan Wasser | 180 °C (Zers.) | 43% |
| 10 | 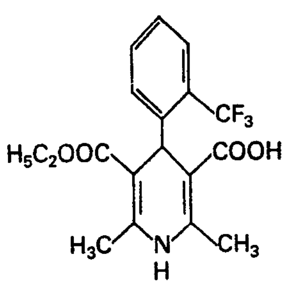 | 1,2-Dimethoxyäthan Wasser | 151 °C (Zers.) | 37% |

Tabelle 1 (Fortsetzung)

| Nr. | Formel | Lösungsmittel | Schmelzpunkt | Ausbeute |
|---|---|---|---|---|
| 11 | 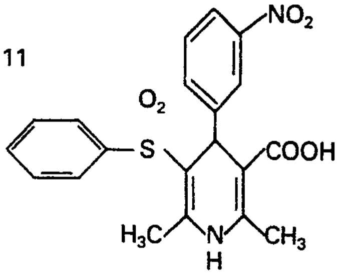 | 1,2-Dimethoxyäthan Wasser | 206 °C (Zers.) | 46% |
| 12 | 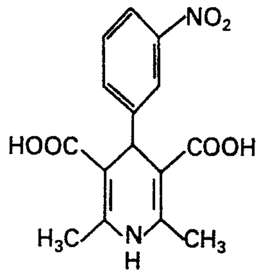 | 1,2-Dimethoxyäthan Wasser | 194 °C (Zers.) | 60% |
| 13 | 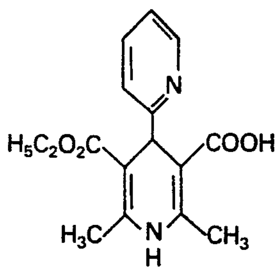 | 1,2-Dimethoxyäthan Wasser | 206 °C (Zers.) | 31% |
| 14 | 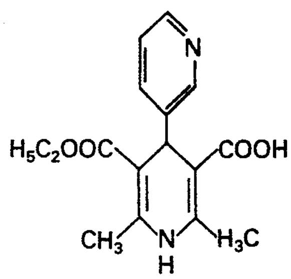 | 1,2-Dimethoxyäthan Wasser | 205 °C (Zers.) | 32% |
| 15 | 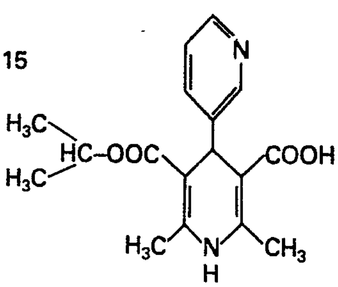 | 1,2-Dimethoxyäthan Wasser | 208 °C (Zers.) | 46% |
| 16 | 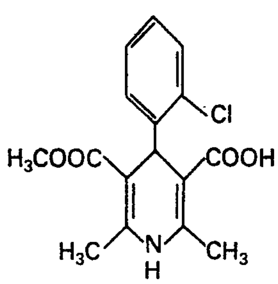 | 1,2-Dimethoxyäthan Wasser | 205 °C (Zers.) | 78% |

**Patentansprüche**

1. Verfahren zur Herstellung von Dihydropyridincarbonsäuren der allgemeinen Formel I

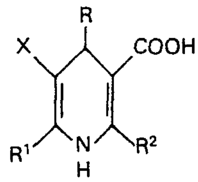

(I)

in welcher

R für einen Pyridylrest oder für einen Phenylrest steht, der ein oder zwei gleiche oder verschiedene Substituenten aus der Gruppe Halogen, Nitro, Cyano, Trifluormethyl, Azido, Trifluormethoxy oder Alkoxy mit 1 bis 2 Kohlenstoffatomen, enthält

$R^1$ und $R^2$ gleich oder verschieden sind und für Wasserstoff, eine Alkyl-Gruppe mit 1 bis 2 Kohlenstoffatomen, einen Phenyl- oder Benzyl-Rest stehen und

a) für die Gruppe –COOH oder

b) für die Gruppe $-COR^3$, wobei $R^3$ Alkyl mit 1 bis 4 Kohlenstoffatomen bedeutet, oder

c) für die Gruppe $-COOR^4$, wobei $R^4$ einen geradkettigen, verzweigten oder cyclischen, gesättigten oder ungesättigten Kohlenwasserstoffrest, der gegebenenfalls durch ein Sauerstoff- oder Schwefelatom oder durch die $-SO_2$-Gruppe in der Kette unterbrochen ist, oder durch 1 bis 2 Trifluormethylgruppen oder durch den Rest

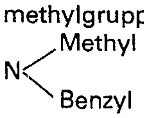 substituiert ist, oder einen Benzylrest

darstellt; oder

d) für die Gruppe $-SO_2-R^5$ steht, wobei $R^5$ einen geradkettigen oder verzweigten, gesättigten oder ungesättigten Kohlenwasserstoffrest mit bis zu 6 Kohlenstoffatomen oder einen Phenylrest bedeutet, dadurch gekennzeichnet, dass man 1,4-Dihydropyridinderivate der allgemeinen Formel II

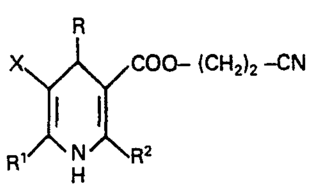

(II)

in welcher

R, $R^1$, $R^2$ und X die oben angegebene Bedeutung haben, in Gegenwart von inerten organischen Lösungsmitteln und Wasser in einem Temperaturbereich von 10–100 °C alkalisch hydrolysiert.

2. Verfahren gemäss Anspruch 1 zur Herstellung von neuen Verbindungen der allgemeinen Formel I, in welcher

R für einen 3-Nitrophenylrest, einen 2-Trifluormethylphenylrest, einen 2-Chlorphenylrest oder einen 2-Methylmercaptopyrridylrest steht,

$R^1$ und $R^2$ für Methyl stehen und

X für die Gruppe –COOH oder $COOR^4$ steht, wobei $R^4$ für einen geradkettigen, verzweigten oder cyclischen Alkylrest mit bis zu 6 Kohlenstoffatomen, der gegebenenfalls durch ein Sauerstoffatom in der Kette unterbrochen oder durch 1 bis 2 Trifluormethylgruppen oder durch den Rest

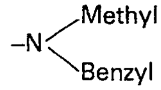 substituiert ist, steht.

3. Verfahren gemäss Anspruch 1, dadurch gekennzeichnet, dass man die Hydrolyse in Gegenwart von Alkalihydroxyden bei Temperaturen zwischen 10 und 100 °C durchführt.

4. Verfahren gemäss Anspruch 1, dadurch gekennzeichnet, dass man die Reaktion bei Temperaturen zwischen 20 und 50 °C mit einem Überschuss von Alkalihydroxyden durchführt.

5. Verfahren gemäss den Ansprüchen 1 bis 4, dadurch gekennzeichnet, dass man als Lösungsmittel niedere aliphatische Alkohole und 1,2-Dimethoxyethan einsetzt.

6. Neue Verbindungen der allgemeinen Formel I

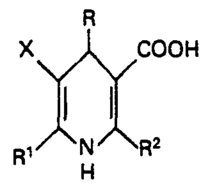

(I)

in welcher

R für einen 3-Nitrophenylrest, einen 2-Trifluormethylphenylrest, einen 2-Chlorphenylrest oder einen 2-Methylmercaptopyrridylrest steht,

$R^1$ und $R^2$ für Methyl stehen und

X für die Gruppe –COOH oder $COOR^4$ steht, wobei $R^4$ für einen geradkettigen, verzweigten oder cyclischen Alkylrest mit bis zu 6 Kohlenstoffatomen, der gegebenenfalls durch ein Sauerstoffatom in der Kette unterbrochen oder durch 1 bis 2 Trifluormethylgruppen oder durch den Rest

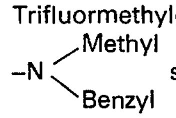 substituiert ist, steht.

7. Verbindungen der allgemeinen Formel I gemäss Anspruch 6 in welcher

R für einen 2-Nitrophenylrest steht,

$R^1$ und $R^2$ jeweils Methyl bedeuten und

X für $COOR^4$ steht, wobei $R^4$ für einen Methyl- oder einen Isobutylrest steht.

8. Verwendung der gemäss Anspruch 1 hergestellten Verbindungen der allgemeinen Formel I zur Herstellung von Dihydropyridin-diestern.

**Revendications**

1. Procédé de préparation d'acides dihydropyridine-carboxyliques de formule générale I

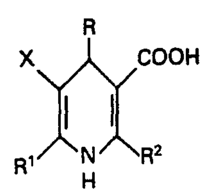

(I)

dans laquelle:

R représente un reste pyridyle ou un reste phényle contenant un ou deux substituants identiques ou différents choisis parmi les atomes d'halogènes, les groupes nitro, cyano, trifluorométhyle, azido, trifluorométhoxy ou alcoxy en $C_1$–$C_2$;

$R^1$ et $R^2$, identiques ou différents, représentent l'hydrogène, un groupe alkyle en $C_1$–$C_2$, un reste phényle ou benzyle et

X représente:

a) le groupe –COOH ou bien

b) le groupe –$COR^3$, dans lequel $R^3$ est un groupe alkyle en $C_1$–$C_4$, ou bien

c) le groupe –$COOR^4$, dans lequel $R^4$ est un reste hydrocarboné saturé ou insaturé, à chaîne droite, ramifiée ou cyclique, qui peut être interrompu le cas échéant dans la chaîne par un atome d'oxygène ou de soufre, ou par le groupe –$SO_2$–, ou substitué par un à deux groupes trifluorométhyle

méthyle
ou par le reste N ou un reste benzyle,
benzyle

ou bien

d) le groupe –$SO_2$–$R^5$, dans lequel $R^5$ est un reste hydrocarboné saturé ou insaturé à chaîne droite ou ramifiée contenant jusqu'à 6 atomes de carbone ou un reste phényle,

caractérisé en ce que l'on soumet à hydrolyse alcaline, dans un intervalle de température de 10 à 100 °C, en présence de solvants organiques inertes et d'eau, des dérivés de 1,4-dihydropyridine de formule générale II

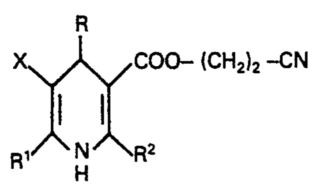

(II)

dans laquelle

R, $R^1$, $R^2$ et X ont les significations indiquées cidessus.

2. Procédé selon la revendication 1 pour la préparation de nouveaux composés de formule générale I dans laquelle

R représente un reste 3-nitrophényle, un reste 2-trifluorométhylphényle, un reste 2-chlorophényle ou un reste 2-méthylmercaptopyridyle,

$R^1$ et $R^2$ représentent des groupes méthyle et

X représente le groupe –COOH ou $COOR^4$ dans lequel $R^4$ est un reste alkyle droit, ramifié ou cyclique, contenant jusqu'à 6 atomes de carbone, qui peut, le cas échéant, être interrompu dans la chaîne par un atome d'oxygène, ou substitué par un à deux groupes trifluorométhyle ou par le reste

méthyle
N
benzyle.

3. Procédé selon la revendication 1, caractérisé en ce que l'on effectue l'hydrolyse en présence d'hydroxydes alcalins à des températures de 10 à 100 °C.

4. Procédé selon la revendication 1, caractérisé en ce que l'on effectue la réaction à des températures de 20 à 50 °C avec un excès d'hydroxydes alcalins.

5. Procédé selon les revendications 1 à 4, caractérisé en ce que l'on utilise en tant que solvants des alcools aliphatiques inférieurs et le 1,2-diméthoxyéthane.

6. Nouveaux composés de formule générale I

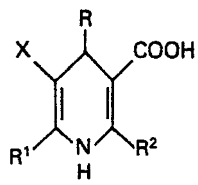

dans laquelle

R représente un groupe 3-nitrophényle, un groupe 2-trifluorométhylphenyle, un groupe 2-chlorophényle ou un groupe 2-méthylmercaptopyridyle,

$R^1$ et $R^2$ représentent des groupes méthyle, et

X représente le groupe –COOH ou $COOR^4$, $R^4$ représentant un groupe alkyle à chaîne droite, ramifiée ou cyclique contenant jusqu'à 6 atomes de carbone, qui peut éventuellement être interrompu dans la chaîne par un atome d'oxygène ou être substitué par un à deux groupes trifluorméthyle ou par le reste

méthyle
–N
benzyle.

7. Composés de formule générale I selon la revendication 6 dans laquelle

R représente un groupe 2-nitrophényle,

$R^1$ et $R^2$ représentent chacun un groupe méthyle, et

X représente $COOR^4$ $R^4$ représentant un groupe méthyle ou isobutyle.

8. Utilisation des composés de formule générale I préparés selon la revendication 1 pour la préparation de diesters de dihydropyridines.

**Claims**

1. Process for the production of dihydropyridinecarboxylic acids of the general formula I

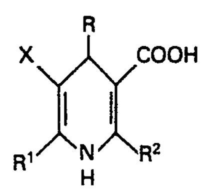

(I)

in which

R represents a pyridyl radical or a phenyl radical which contains one or two identical or different substituents from the group of halogen, nitro, cyano, trifluoromethyl, azido, trifluoromethoxy or alkoxy with 1 to 2 carbon atoms,

$R^1$ and $R^2$ are identical or different and represent hydrogen, an alkyl group with 1 to 2 carbon atoms, a phenyl radical or a benzyl radical and

X a) represents the group –COOH or

b) represents the group –$COR^3$, wherein $R^3$ denotes alkyl with 1 to 4 carbon atoms, or

c) represents the group –$COOR^4$, wherein $R^4$ represents a straight-chain, branched or cyclic, saturated or unsaturated hydrocarbon radical which is optionally interrupted in the chain by an

oxygen or sulphur atom or by the $-SO_2-$ group or which is substituted by 1 to 2 trifluoromethyl groups or by the radical

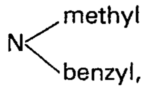
or represents a benzyl radical, or

d) represents the group $-SO_2-R^5$, wherein $R^5$ denotes a straight-chain or branched, saturated or unsaturated hydrocarbon radical with up to 6 carbon atoms, or a phenyl radical,

characterised in that 1,4-dihydropyridine derivatives of the general formula II

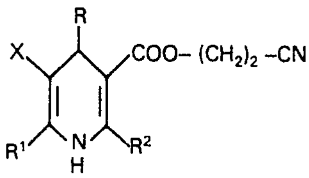
(II)

in which

R, $R^1$, $R^2$ and X have the meaning indicated above, are hydrolysed under alkaline conditions in the presence of inert organic solvents and water in a temperature range from 10 to 100 °C.

2. Process according to Claim 1 for the preparation of new compounds of the general formula I in which

R represents a 3-nitrophenyl radical, a 2-trifluoromethylphenyl radical, a 2-chlorophenyl radical or a 2-methylmercaptopyrridyl radical,

$R^1$ and $R^2$ represent methyl and

X represents the group –COOH or $COOR^4$, wherein $R^4$ represents a straight-chain, branched or cyclic alkyl radical with up to 6 carbon atoms, which is optionally interrupted by an oxygen atom in the chain or is substituted by 1 to 2 trifluoromethyl groups or by the radical

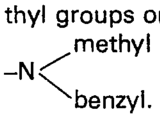

3. Process according to Claim 1, characterised in that the hydrolysis is carried out in the presence of alkali metal hydroxides at temperatures between 10 and 100 °C.

4. Process according to Claim 1, characterised in that the reaction is carried out at temperatures between 20 and 50 °C with an excess of alkali metal hydroxides.

5. Process according to Claims 1 to 4, characterised in that low aliphatic alcohols and 1,2-dimethoxyethane are used as solvents.

6. New compounds of the general formula I

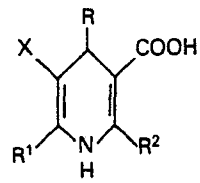
(I)

in which

R represents a 3-nitrophenyl radical, a 2-trifluoromethylphenyl radical, a 2-chlorophenyl radical or a 2-methylmercaptopyrridyl radical,

$R^1$ and $R^2$ represents methyl and

X represents the group –COOH or $COOR^4$, wherein $R^4$ represents a straight-chain, branched or cyclic alkyl radical with up to 6 carbon atoms, which is optionally interrupted by an oxygen atom in the chain or is substituted by 1 to 2 trifluoromethyl groups or by the radical

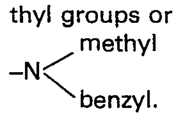

7. Compounds of the general formula I according to Claim 6 in which

R represents a 2-nitrophenyl radical,

$R^1$ and $R^2$ each denote methyl and

X represents $COOR^4$, wherein $R^4$ represents a methyl or an isobutyl radical.

8. Use of the compounds of the general formula I, prepared according to Claim 1, for the production of dihydropyridine diesters.